# EUROPEAN PATENT SPECIFICATION

(11) **EP 2 836 813 B1**
(45) Date of publication and mention of the grant of the patent: **26.12.2018**
(21) Application number: 13776001.3
(22) Date of filing: 05.04.2013
(51) Int. Cl.: G01N 1/28, H05H 1/00

(54) **PLASMA TREATMENT FOR DNA BINDING**
PLASMABEARBEITUNG ZUR DNS-BINDUNG
TRAITEMENT PLASMA POUR LIER UN ADN

(30) Priority: 09.04.2012 US 201261621739 P; 15.03.2013 US 201313836238
(43) Date of publication of application: 18.02.2015
(73) Proprietor: APDN (B.V.I.) Inc., Road Town Tortola (VG)
(72) Inventor: BERRADA, Abdelkrim, Lake Rononkoma, NY 11779 (US); LIANG, MingHwa, Benjamin, East Setauket, NY 11733 (US); HAYWARD, James, Arthur, Stony Brook, NY 11790 (US); JUNG, Lawrence, Forest Hills, NY 11375 (US)
(74) Representative: HGF Limited
(86) International application number: PCT/US2013/035477
(87) International publication number: WO 2013/154943

(56) References cited:
- EP-A1- 1 063 286
- EP-A1- 1 231 470
- EP-A1- 1 847 316
- WO-A1-2006/109014
- WO-A1-2008/154931
- WO-A2-2005/103226
- WO-A2-2007/078833
- US-A- 6 013 789
- US-A1- 2002 128 234
- US-A1- 2002 155 490
- US-A1- 2005 142 565
- US-A1- 2008 153 135
- KANEDA S. ET AL.: 'Modification of the glass surface property in PDMS-glass hybrid microfluidic devices.' ANALYTICAL SCIENCES vol. 28, no. 1, January 2012, pages 39 - 44, XP055171221
- HOSOKAWA K. ET AL.: 'DNA detection on a power-free microchip with laminar flow-assisted dendritic amplification.' ANALYTIC SCIENCES vol. 26, no. 10, 2010, pages 1053 - 1057, XP055171223
- PARK H. ET AL.: 'Stress response of fibroblasts adherent to the surface of plasma- treated poly(lactic-co-glycolic acid) nanofiber matrices' COLLOIDS SURF B BIOINTERFACES vol. 77, no. 1, 01 May 2010, pages 90 - 95, XP026935199
- TUZLAKOGLU K. ET AL.: 'A new route to produce starch-based fiber mesh scaffolds by wet spinning and subsequent surface modification as a way to improve cell attachment and proliferation' J BIOMED MATER RES A vol. 92, no. 1, January 2010, pages 369 - 77, XP055171225
- ZIMMER ET AL: "Alkaline denaturation of DNA's from various sources", BIOCHIMICA ET BIOPHYSICA ACTA, N. NUCLEIC ACIDS AND PROTEINSYTHESIS, AMSTERDAM, NL, vol. 161, no. 2, 1 January 1968 (1968-01-01), pages 584-586, XP023367206, ISSN: 0005-2787, DOI: 10.1016/0005-2787(68)90141-X [retrieved on 1968-01-01]
- M. Ageno ET AL: "The Alkaline Denaturation of DNA", Biophysical Journal, vol. 9, no. 11, 1 November 1969 (1969-11-01), pages 1281-1311, XP055296034, AMSTERDAM, NL ISSN: 0006-3495, DOI: 10.1016/S0006-3495(69)86452-0
- DAVISON ET AL: "The rate of strand separation in alkali-treated DNA", JOURNAL OF MOLECULAR BIOLOGY, ACADEMIC PRESS, UNITED KINGDOM, vol. 22, no. 1, 15 December 1966 (1966-12-15), pages 97-108, XP024011505, ISSN: 0022-2836, DOI: 10.1016/0022-2836(66)90182-3 [retrieved on 1966-12-15]
- STUDIER ET AL: "Sedimentation studies of the size and shape of DNA", JOURNAL OF MOLECULAR BIOLOGY, ACADEMIC PRESS, UNITED KINGDOM, vol. 11, no. 2, 1 February 1965 (1965-02-01), pages 373-390, XP026102213, ISSN: 0022-2836, DOI: 10.1016/S0022-2836(65)80064-X [retrieved on 1965-02-01]
- CROTHERS ET AL: "The kinetics of DNA denaturation", JOURNAL OF MOLECULAR BIOLOGY, ACADEMIC PRESS, UNITED KINGDOM, vol. 9, no. 3, 1 January 1964 (1964-01-01) , pages 712-733, XP026102315, ISSN: 0022-2836, DOI: 10.1016/S0022-2836(64)80177-7 [retrieved on 1964-01-01]

## Description

### TECHNICAL FIELD

The invention relates to a composition, a method of binding DNA to plasma-treated surfaces, and a system for binding DNA to treated surfaces.

### DISCUSSION OF THE RELATED ART

Plasma, often called the fourth state of matter, is a gas-like state in which a certain portion of the particles are ionized. In particular, a plasma is an electrically neutral medium of positive and negative particles, in which the overall charge of a plasma is approximately zero. Although the particles of a plasma are unbound, these particles are not totally free, as their translational motion generates electrical currents and magnetic fields, which affect each other. Because of this electrical conductivity, plasmas are distinct from other lower-energy states of matter, such as solids, liquids, and gases. Although plasma is closely related to the gas phase in that it has no definite form or volume, it differs by frequently having a non-Maxwellian velocity distribution, and in the nature of the particle interactions. Plasmas are by far the most common phase of matter in the universe, both by mass and by volume. All stars are made of plasma, and even the interstellar space is filled with plasma.

An example of a plasma is the solar wind, a stream of ions continuously flowing outward from the Sun. The Earth's magnetic field traps these particles, many of which travel toward the poles where they are accelerated toward Earth. Collisions between these ions and atmospheric atoms and molecules cause energy releases in the form of the *aurora borealis* and the *aurora australis* appearing over the north and south poles, respectively and are the source of the erratic interference in radio reception.

The degree of ionization of a plasma is the proportion of atoms that have lost or gained electrons, and is determined primarily by the temperature. Even a partially ionized gas in which as few as 1% of the particles are ionized can behave as a plasma. The temperature of a plasma is a measure of the average thermal kinetic energy per particle. The degree of plasma ionization is determined by the electron temperature relative to the ionization energy. In many cases the electrons in a plasma are close enough to thermal equilibrium that their temperature is relatively well-defined, even when there is a significant deviation from a Maxwellian energy distribution. Because of the large mass differences between electrons and ions and neutral atoms, electrons come to thermodynamic equilibrium among themselves much faster than they come into equilibrium with the ions or neutral atoms. For this reason, the ion temperature may be very different from, and is usually lower than the electron temperature.

Based on the relative temperatures of the electrons, ions and neutrals, plasmas are classified as "thermal" or "non-thermal". Thermal plasmas are in thermal equilibrium, so that the electrons and the heavy particles have the same temperature. On the other hand, in non-thermal plasmas, the ions and particles have a much lower temperature than the electrons. Non-thermal, non-equilibrium plasmas are typically not as ionized as thermal plasmas, and have lower energy densities, and thus the temperature is not dispersed uniformly among the particles. A plasma is sometimes referred to as being "hot" if it is almost fully ionized, and "cold" if only a small fraction of the gas molecules are ionized. However, even in a "cold" plasma, the electron temperature is still typically several thousand degrees Kelvin.

A plasma may be produced by heating a gas to ionize its molecules or atoms, e.g. in a flame to produce a flame-based plasma, or by applying strong electromagnetic fields, e.g., by using a laser or microwave generator. However, all methods of producing a plasma require the input of energy to produce and sustain it. For example, a plasma can be generated when an electrical current is applied across a dielectric gas or fluid in a discharge tube. The potential difference and subsequent electric field pull the bound, negative, electrons toward the anode while the cathode pulls the nuclei. As the voltage increases, the current electrically polarizes the material beyond its dielectric limit into a stage of electrical breakdown, and the material transforms from an insulator into a conductor as it becomes increasingly ionized. Collisions between electrons and neutral gas atoms create more ions and electrons, and the number of charged particles increases rapidly after about 20 successive sets of collisions due to the small mean free path.

Plasmas are useful in industrial manufacturing for cleaning sensitive products such as computer chips and other electronic components. Plasma cleaning involves the removal of impurities and contaminants from surfaces through the application of an energetic plasma. These treatment systems use electric fields to direct reactive gases toward the surface. Low molecular weight materials such as water, absorbed gases and polymer fragments are knocked off the surface to expose a clean, uncontaminated surface. At the same time a percentage of the reactive components in the plasma bond to the freshly exposed surface, changing the chemistry of the surface and imparting the desired functionalities. Gases such as argon and oxygen, as well as mixtures such as air and hydrogen/nitrogen can be used. The plasma can be produced by using a high frequency voltage (typically kHz to MHz) to ionize a gas at low pressure (e.g. at one thousandth of atmospheric pressure or lower, i.e. in a vacuum) or alternatively, the plasma can be produced at atmospheric pressure. The plasma includes atoms, molecules, ions, electrons, free radicals, and photons in the short wave ultraviolet (vacuum UV, or VUV for short) range. This mixture, which can be at room temperature, then interacts with any surface placed in the plasma.

If the gas used is oxygen, the plasma is an effective, economical, environmentally safe method for critical cleaning. The VUV energy can break most organic bonds of surface contaminants to disrupt high molecular weight contaminants. A second cleaning action can be carried out using the highly reactive oxygen species (O₂⁺, O₂⁻, O₃, O, O⁺, O⁻, ionized ozone, metastable excited oxygen, and free electrons) produced in the plasma. These species react with organic contaminants to form H₂O, CO, CO₂, and low molecular weight hydrocarbons which have relatively high vapor pressures and are easily evacuated from low pressure chambers during processing. The resulting surface is ultra-clean.

If the surface to be treated consists of easily oxidized materials such as silver or copper, inert gases such as argon or helium can be used instead to avoid these reactions at the treated surface. The treated atoms and ions behave like a molecular sandblast and can break down organic contaminants. These contaminants are again vaporized and can be evacuated in a low pressure chamber.

Plasmas have many industrial applications, including, without limitation, industrial and extractive metallurgy, surface treatments such as thermal spraying, etching in microelectronics, metal cutting and welding, as well as in everyday vehicle exhaust cleanup and fluorescent/luminescent lamps, while even having a role in supersonic combustion engines for aerospace engineering. The present invention relates to the use of these plasma properties for the preparation of surfaces for DNA binding.

DNA (deoxyribonucleic acid) can exist as an unstructured single strand or as a double-stranded helix composed of nucleotides linked in chains through phosphodiester bonds. The nucleotides that make up the DNA are composed of nitrogen-containing "bases" conjugated to a pentose sugar phosphate ester chain or backbone. There are four naturally occurring bases in DNA, two are purines: adenine and guanine, conventionally represented as A and G, respectively, and two are pyrimidines: thymine and cytosine, conventionally represented as T and C. Adenine bases of the nucleotide chain have a natural affinity for pairing to thymine bases due to the two hydrogen bonds formed between the A:T base pairs, and guanine bases pair with cytosine even more strongly by virtue of the three hydrogen bonds formed between them. Each of the pentose sugars in the DNA chain is a 2'-deoxyribose bonded to the two neighboring nucleotides in the DNA by phosphate groups at the 5' and the 3' positions of the pentose ring forming the sugar-phosphate backbone. Each deoxyribose sugar is also covalently bonded to a nitrogenous base at the 1' position of the ribose of the sugar-phosphate backbone of the polynucleotide chain.

The combination of bases can be arranged in any order or sequence in each individual strand, however, the base sequences of the two strands of double-stranded DNA are not identical, but rather are complementary. The strands are anti-parallel, meaning that 5' to 3' orientations of the two strands run in parallel, but opposite directions in the two strands. Also, each base of one strand is hydrogen bonded to its complementary base on the other strand, adenine pairing with thymine, and guanine pairing with cytosine. Each oligonucleotide or polynucleotide strand has a free 5' terminal phosphate and a free 3' terminal hydroxyl group. These free 5' and 3' terminal groups of the oligonucleotide or polynucleotide and are available for reaction with chemically reactive functional groups, such as functional groups of a plasma activated surface of a substrate or object.

DNA found in the genomes of living organisms encodes the biological information of the organism and can be thought of as the blueprint for the particular animal, plant, fungus or bacterium. The diversity of animal and plant life is a testament to the vast coding capacity and the stability of information encoded in DNA molecules.

The immense informational coding capacity and conservation of informational sequences in DNA renders these molecules useful for other purposes, such as for instance as a unique marker or "taggant" for an object to which it can be bound. This can be for identification purposes, such as for instance in quality assurance and quality control (QA & QC), or for authentication or verification of valuable items which cannot be easily copied and thus protect against counterfeits that may be of lower quality than the authentic item and erode the market for the genuine article.

Minute quantities of DNA can be detected by a variety of physical methods after amplification. In principle, a single DNA strand can be serially duplicated in a polymerase chain reaction (PCR) by techniques well known in the art to produce detectable amounts of double stranded copies that can be quantified and if desired, subjected to DNA sequencing for verification purposes. See for example, D'Haene B., et al. Accurate and objective copy number profiling using real-time quantitative PCR. Methods. 2010, 50(4):262-70; Kubista M, et al. The real-time polymerase chain reaction. Mol Aspects Med. (2006) 27(2-3):95-125; Righettia, P.G. and Gelfib, C. Recent Advances in Capillary Electrophoresis of DNA Fragments and PCR Products in Poly(N-substituted Acrylamides) Analytical Biochemistry (1997) 244(2): 195-207.

US6013789 describes immobilization of oligonucleotides to a plasma-treated polypropylene surface for hybridization assays. WO2005/103226 describes the treatment of different surfaces like glass, stainless steel, quartz, or oxidized silicon surface with plasma and the use thereof for binding DNA. EP1847316 describes a polymeric solid support for microarrays which is treated with plasma in order to improve binding of different molecules, inter alia of DNA.

### SUMMARY

The present invention provides a composition comprising alkaline-activated single stranded marker DNA bonded to a plasma-treated surface wherein the composition is produced by applying alkaline-activated DNA to the plasma treated surface.

In one embodiment, the plasma treatment comprises treatment with a plasma selected from an argon plasma, a compressed air plasma, a flame-based plasma and a vacuum plasma.

In one embodiment, the surface is a surface of a ceramic, a semiconductor, a metal, a fabric or an organic polymer.

In one embodiment, the DNA consists essentially of from about 20 to about 10,000 bases

In one embodiment, the DNA consists essentially of from about 50 to about 5,000 bases.

In one embodiment, the DNA consists essentially of from about 75 to about 500 bases.

The invention further provides a method of binding DNA to a surface, wherein the method includes the steps of exposing the surface to a plasma to produce a plasma-treated surface; exposing DNA to alkaline conditions; and applying the alkaline-activated DNA to the plasma-treated surface, to produce surface bound DNA on the plasma-treated surface.

In one embodiment, the method further comprises extracting a DNA sample from the plasma-treated surface and amplifying the extracted DNA sample to produce an amplified DNA sample;
wherein the amplified DNA sample is identified as the DNA applied to the plasma-treated surface.

In one embodiment, the extracted DNA sample is amplified using PCR.

In one embodiment, plasma-treated surface comprises reactive functional groups created on the surface by the plasma treatment.

In one embodiment, a concentration of DNA applied to the treated surface is at least about one femtogram per liter (∼10⁻¹⁵g/L).

The invention also provides a system for binding DNA to a surface, comprising a plasma generator adapted to treating a surface with a plasma to produce a plasma-treated surface; and an applicator containing alkaline-activated single stranded marker DNA adapted to applying DNA to the plasma-treated surface, to produce surface bound DNA on the plasma-treated surface.

In one embodiment, the DNA consists essentially of from about 25 to about 10,000 bases.

In one embodiment, the DNA consists essentially of from about 50 to about 5,000 bases.

In one embodiment, the DNA consists essentially of from about 75 to about 500 bases.

In addition, the disclosure further provides a method of binding an alkali-treated DNA to a surface, wherein the method includes the steps of exposing the surface to a plasma to produce a plasma-treated surface; and applying an alkali treated DNA to the plasma-treated surface, to produce surface bound DNA on the plasma-treated surface.

### BRIEF DESCRIPTION OF THE FIGURES

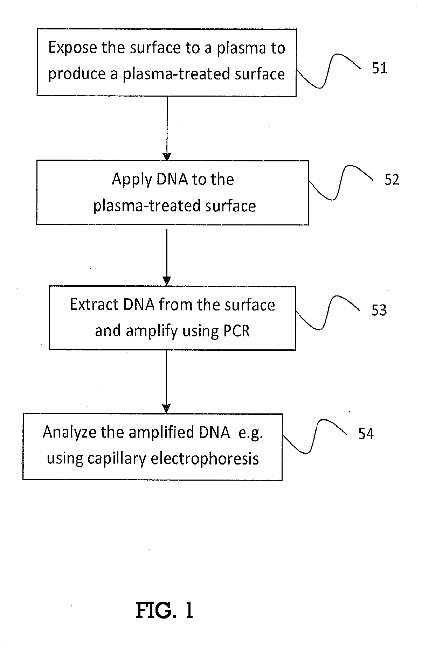

FIG. 1 is a flowchart of a method for binding DNA to a surface of an object, according to the present disclosure.

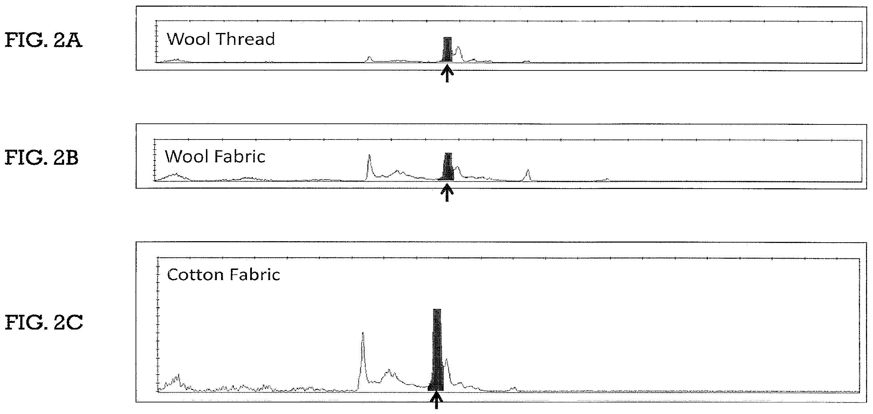

FIGS. 2A, 2B and 2C show DNA detection results obtained with samples from wool thread, wool fabric and cotton fabric, respectively, each of which had been plasma-treated before applying the marker DNA.

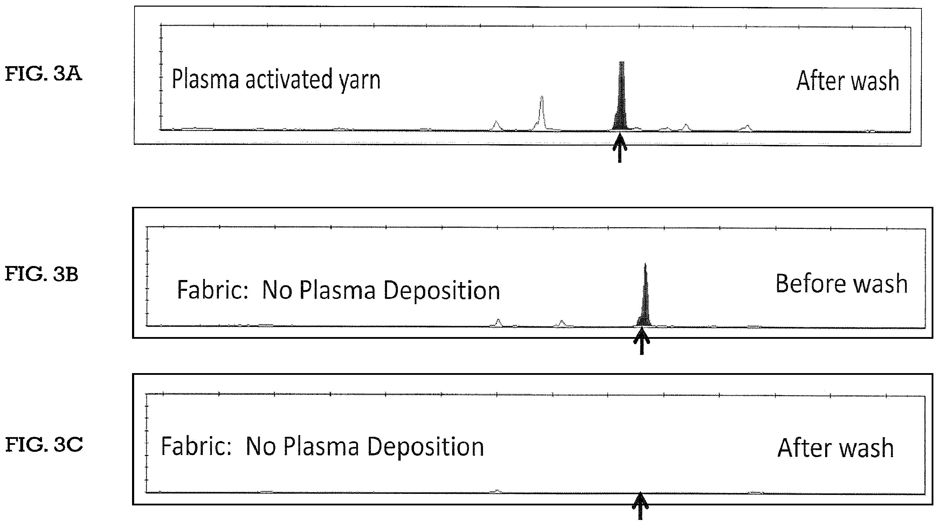

FIG. 3A shows DNA detection results obtained with samples from a yarn after washing the DNA bound to the yarn that had been plasma-treated. FIGS. 3B and 3C show DNA detection results obtained with DNA extracted from a fabric swatch before and after washing the DNA bound to the surface that had not been plasma-treated.

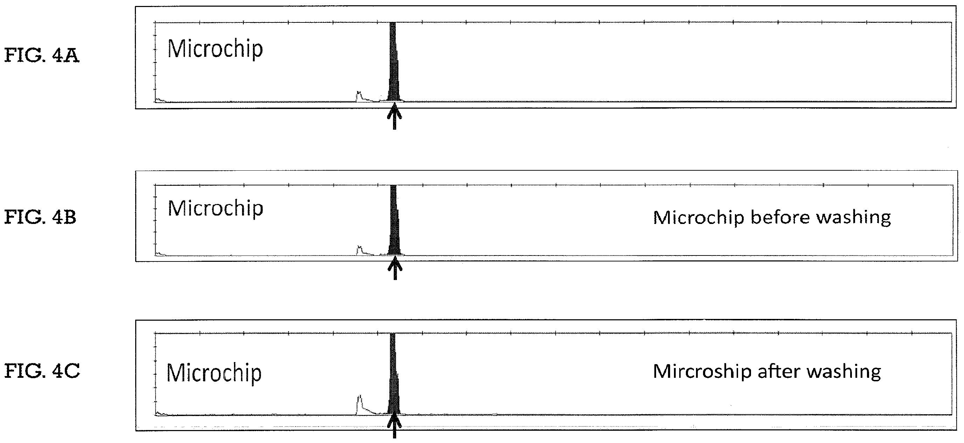

FIG. 4A shows a representative result of DNA detection from a microchip after DNA application onto a plasma treated surface of the microchip. FIGS. 4B and 4C show DNA detection results obtained with DNA extracted from a metal microchip before and after washing. The DNA had been applied after the surface of the microchip had been plasma-treated.

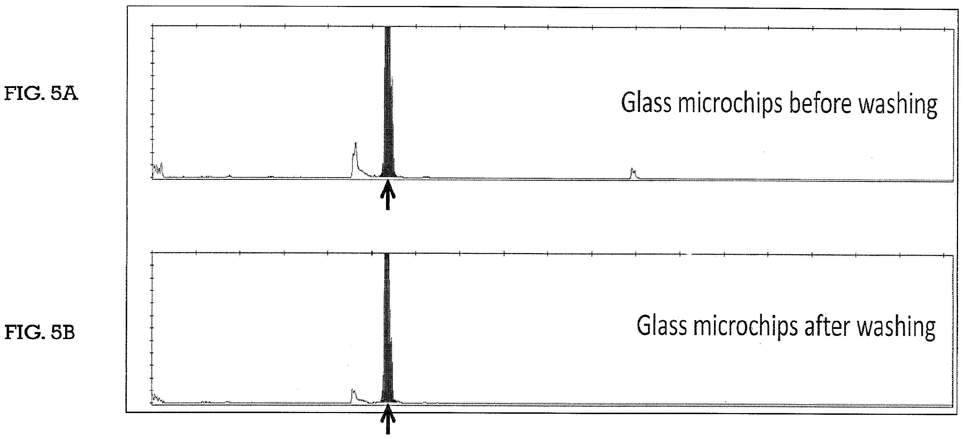

FIGS. 5A and 5B: DNA detection results with DNA extracted from glass microchip before and after washing DNA bound to plasma-treated surface.

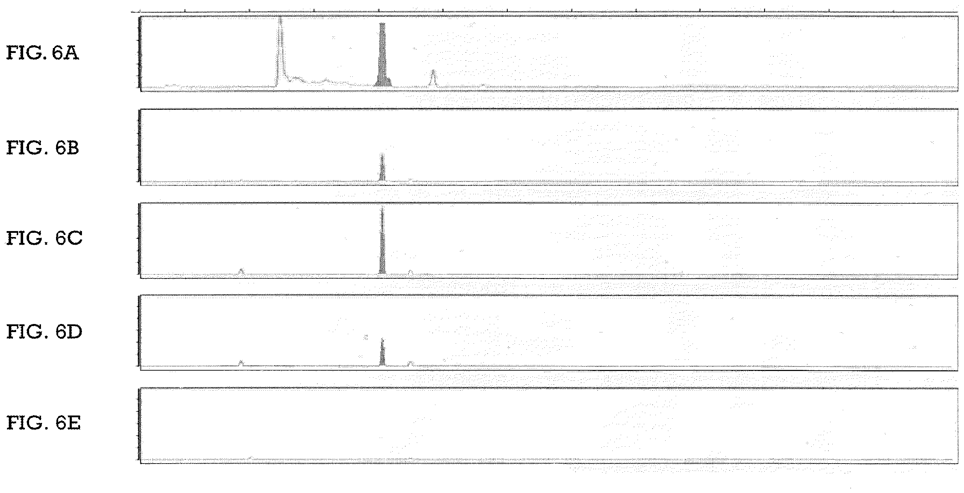

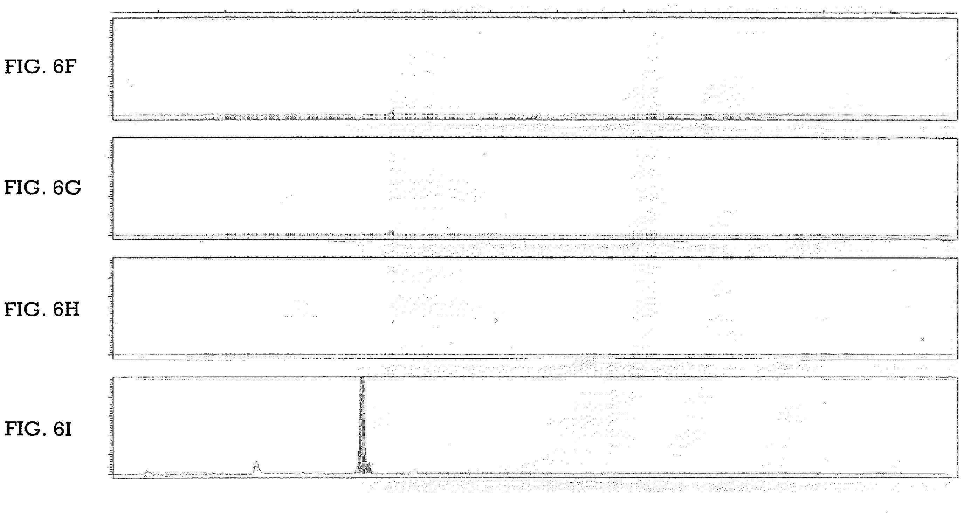

FIGS. 6A-6I: Capillary electrophoresis results with plasma-treated foil. FIG. 6A: Aluminum foil clipping after DNA binding to plasma-treated foil. FIG. 6B: Clipping after running tap water wash. FIG. 6C: 1 uL sample of a 50 ml water wash from first serial wash. FIG. 6D: 1 uL sample from second serial wash. FIG. 6E: 1 uL sample from third serial wash. FIG. 6F: 1 uL sample from fourth serial wash. FIG. 6G: 1 uL sample from fifth serial wash. FIG. 6H: 1 uL sample of from sixth serial wash. FIG. 6I: Clipping after sixth serial wash.

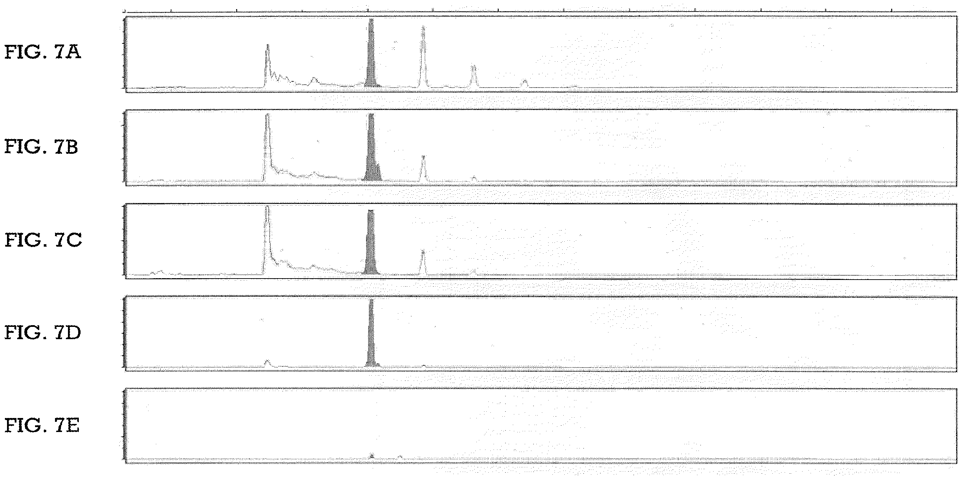

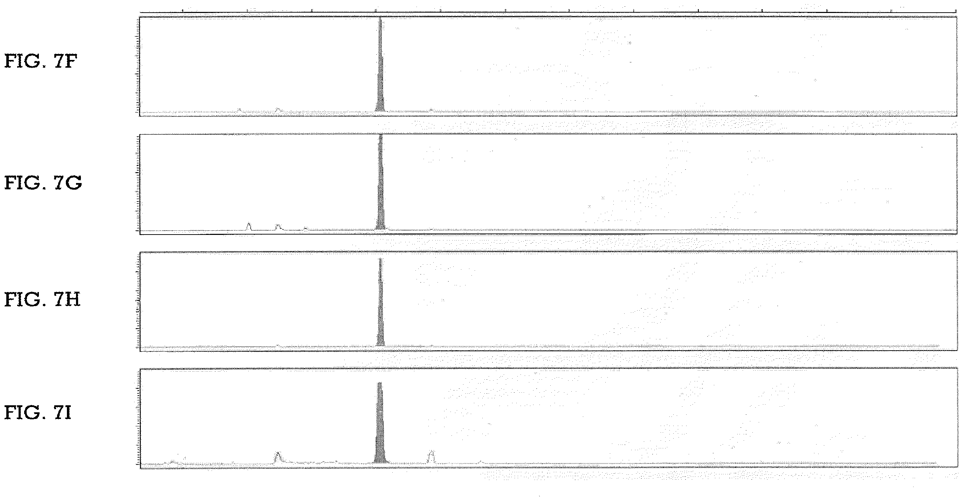

FIGS. 7A-7I show capillary electrophoresis results obtained with aluminum foil without plasma treatment. FIG. 7A: Aluminum foil clipping after DNA binding to foil with no plasma-treatment. FIG. 7B: Clipping after wash under running tap water. FIG. 7C: 1 uL sample from first serial wash. FIG. 7D: 1 uL sample from second serial wash. FIG. 7E: 1 uL sample from third serial wash. FIG. 7F: 1 uL sample from fourth serial wash. FIG. 7G: 1 uL sample from fifth serial wash. FIG. 7H: 1 uL sample from sixth serial wash. FIG. 7I: Clipping after sixth serial wash.

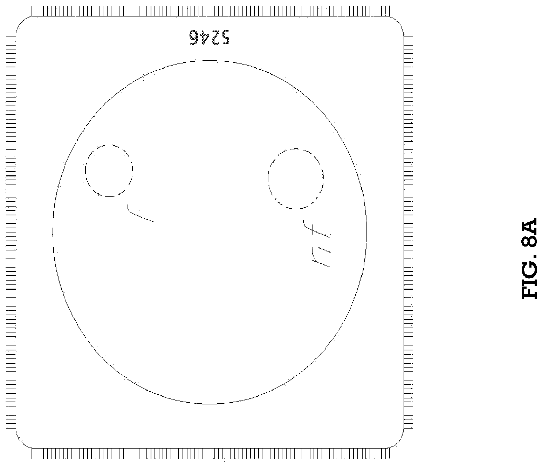

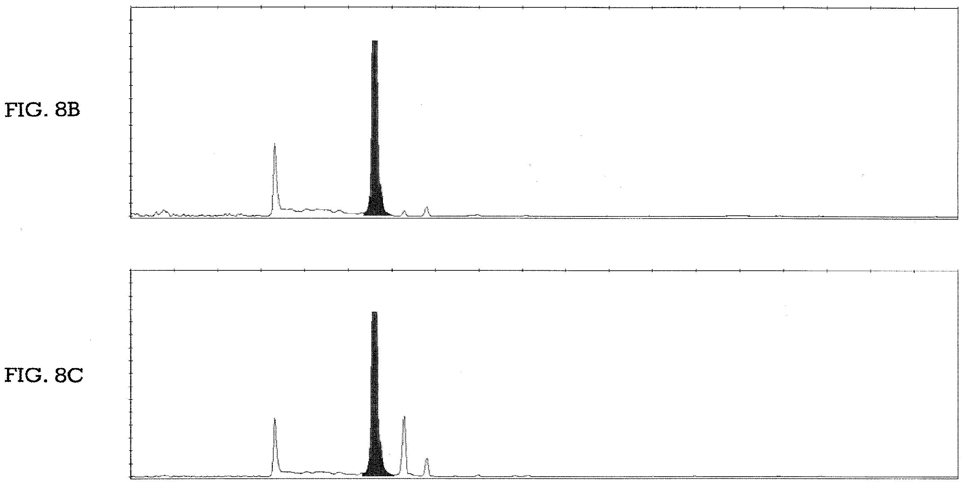

FIG. 8A: Image of the microchip labeled with marker DNA and fluorophore (F) in the top spot, or marker DNA with no fluorophore (nf) in the bottom spot. Spots are outlined by a dashed line. FIG. 8B: Capillary electrophoresis results from an amplification of ethanol swab sample from DNA spot on the plastic/ceramic portion of the microchip. FIG. 8C: Capillary electrophoresis results from an amplification of ethanol swab sample from DNA spot on the metallic portion of the microchip.

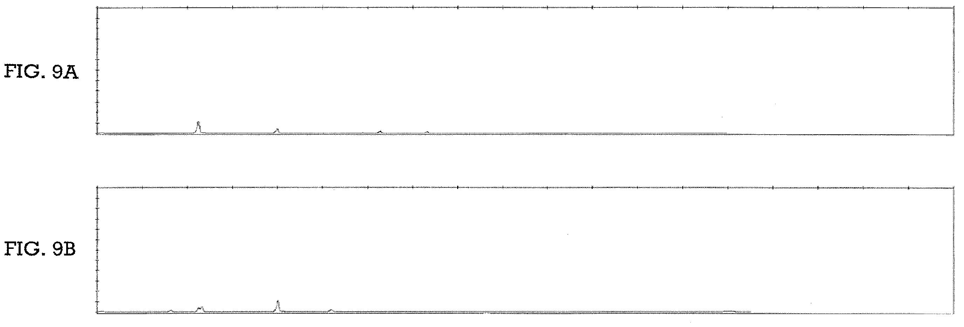

FIGS. 9A and 9B: capillary electrophoresis results from amplifications of ethanol swab samples from fingertips of latex glove after rubbing against the marker DNA spot applied to a plasma-treated microchip surface.

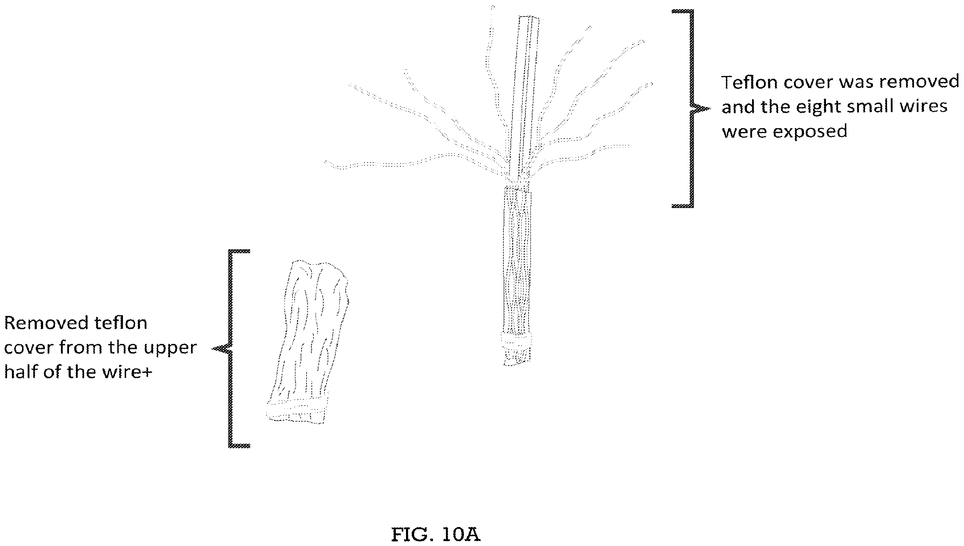

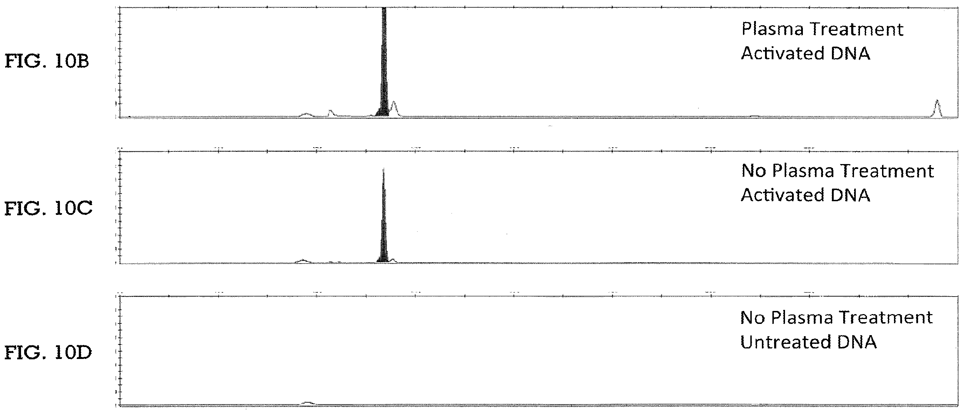

FIG. 10A: Partially exposed metal wire with a Teflon® sheath removed from a portion of the wire on a disposable bench pad. FIG. 10B: Capillary electrophoresis results from amplification of sample of alkali activated DNA-treated exposed wire that had been plasma pretreated. FIG. 10C: Capillary electrophoresis results from amplification of sample of alkali activated DNA-treated exposed wire without plasma pretreatment. FIG. 10D: Capillary electrophoresis results from amplification of sample of marker DNA-treated exposed wire with no plasma pretreatment.

### DETAILED DESCRIPTION

### Definitions:

As used herein, the terms "binding to a substrate" and "immobilizing" are interchangeable as applied to DNA binding and immobilization.

The term "taggant" as used herein denotes a DNA marker, and optionally the DNA marker can be in combination with a second marker substance. The marker DNA and the additional one or more markers, when present are affixed to an object to indicate a property of the object, such as for instance its source of manufacture. The object to be marked with the taggant can be any solid traceable item, such as an electronic device, an item of clothing, paper, fiber, or fabric, or any other item of commerce, or cash or valuables, whether in storage or in transit. Alternatively, the item of commerce to be marked with the taggant can be a liquid, such as for instance an ink, a dye or a spray. In another alternative, the item of commerce can be a commodity item, such as paper, metal, wood, a plastic or a powder. The taggant can be, for example, specific to the company or the type of item (e.g. a model number), specific to a particular lot or batch of the item (lot number), or specific to the actual item, as in, for instance, a serial number unique to the item. In addition, the taggant can indicate any one or more of a variety of other useful items of data; for example, the taggant can encode data that indicates the name and contact information of the company that manufactured the tagged product or item, the date of manufacture, the distributor and/or the intended retailer of the product or item. The taggant can also indicate, for example and without limitation, component data, such as the source of the component incorporated into the item or the identity of the production plant or machinery that was used in the manufacture of the product or item; the date that the product or item was placed into the stream of commerce, the date of acceptance by the distributor and/or the date of delivery to the retailer and any other useful commercial, or other data such as for instance personal information of the owner of a custom made item. Each element of data or indicia can be encrypted or encoded in the taggant and can be deciphered from taggant recovered from the object and decoded or decrypted according to the methods described herein. The decoded or decrypted data can then be used to verify the properties of the object, or to authenticate the object, or to exclude counterfeit items.

The term "PCR" refers to a polymerase chain reaction. PCR is an amplification technology useful to expand the number of copies of a template nucleic acid sequence via a temperature cycling through melting, re-annealing and polymerization cycles with pairs of short primer oligonucleotides complementary to specific sequences bordering the template nucleic acid sequence in the presence of a DNA polymerase, preferably a thermostable DNA polymerase such as the thermostable *Taq* polymerase originally isolated from the thermophillic bacterium (*Thermus aquaticus).* PCR includes but is not limited to standard PCR methods, where in DNA strands are copied to provide a million or more copies of the original DNA strands (e.g. PCR using random primers: See for instance PCR with Arbitrary Primers: Approach with Care. W.C. Black IV, Ins. Mol. Biol. 2: 1-6, Dec. 2007); Real-time PCR technology, wherein the amount of PCR products can be monitored at each cycle (Real time quantitative PCR: C.A. Heid, J. Stevens, K.J. Livak and P.M. Williams, 1996 Genome Research 6: 986-994); Reverse transcription PCR wherein RNA is first copied in DNA stands and thereafter the DNA strands are amplified by standard PCR reactions (See for example: Quantitative RT-PCR: Pitfalls and Potential: W.F. Freeman, S.J. Walker and K.E. Vrana; BioTechniques 26:112-125, January 1999).

The term "monomer" as used herein refers to any chemical entity that can be covalently linked to one or more other such entities to form an oligomer or a polymer. Examples of "monomers" include nucleotides, amino acids, saccharides, amino acids, and the like.

The term "nucleic acid" means a polymer composed of nucleotides which can be deoxyribonucleotides or ribonucleotides. These compounds can be natural or synthetically produced deoxyribonucleotides or ribonucleotides. The synthetically produced nucleic acid can be of a naturally occurring sequence, or a non-natural unique sequence.

The terms "ribonucleic acid" and "RNA" denote a polymer composed of ribonucleotides. The terms "deoxyribonucleic acid" and "DNA" denote a polymer composed of deoxyribonucleotides.

The term "nucleotide" means a monomeric unit comprising a sugar phosphate, usually ribose-5'-phosphate or 2'-deoxyribose-5'-phosphate covalently bonded to a nitrogen-containing base, usually, adenine (A), guanine (G), cytosine (C), or thymine (T) in the case of a deoxyribonucleotide, and usually, adenine (A), guanine (G), cytosine (C), or uracil (U) in the case of ribonucleotides.

The term "oligonucleotide" as used in this specification refers to single or double stranded polymer composed of covalently nucleotide monomers forming a chain of from two to about twenty nucleotides in length.

The term "polynucleotide" as used in this specification refers to single or double stranded polymer composed of covalently nucleotide monomers forming a chain of generally greater than about twenty nucleotides in length.

Nucleic acids having a naturally occurring sequence can hybridize with nucleic acids in a sequence specific manner. That is they can participate in hybridization reactions in which the complementary base pairs A:T (adenine:thymine) and G:C (guanine:cytosine) form intermolecular (or intra-molecular) hydrogen bonds and cooperative stacking interactions between the planar neighboring bases in each strand through Pi electrons, together known as Watson-Crick base pairing interactions. The bases of the nucleic acid strands can also hybridize to form non-Watson-Crick base pairs by so-called "wobble" interactions in which G (guanine) pairs with U (uracil), or alternatively, I (inosine) pairs with C (cytosine), U (uracil) or A (adenine), but with lower binding energies than the normal Watson-Crick base pairing interactions.

The term "identifiable sequence" or "detectable sequence" means a nucleotide sequence which can be detected by hybridization and/or PCR technology by a primer or probe designed for specific interaction with the target nucleotide sequence to be identified. The interaction of the target nucleotide sequence with the specific probe or primer can be detected by optical and/or visual means to determine the presence of the target nucleotide sequence.

The disclosure provides a method by which DNA and fluorophore can be bound to various substrates that have been plasma-treated. With this method DNA can be bound to materials, resist all kinds of finishing processes, such as washing and cleaning, and yet be safely retrieved in order to authenticate the product. Authentication can occur by several methods. One method involves adding fluorophore to the product, making rapid identification possible, as a UV light could detect the presence of a fluorophore. Another authentication method involves binding DNA to substrates via a chemical linker. A linker often includes a chain of carbon atoms with a reactive functional group at the end. This reactive functional group can be activated to bind covalently to an available group or to the substrate or the product to be marked. This DNA attached to the product is unique to the particular product and therefore acts as its fingerprint, making authentication possible. These methods combined would create a fool proof method of identification, where the fluorescence of the product would be the first level of protection and the DNA would be the second, unique and definite layer that could not be duplicated.

The disclosure provides botanical DNA markers, SigNature™ DNA (Applied DNA Sciences, Stony Brook, NY) that essentially cannot be copied by would-be counterfeiters, and are resistant to various chemical and textile treatments. To ensure adherence, SigNature™ DNA was formulated to be tightly bound to both natural and synthetic fibers and other amorphous material such as wool, cotton, polyesters, such as for instance, nylon and polyethylene terephthalate (PET). These textile fabrics can be marked with SigNature™ DNA during the manufacturing process circumventing the need for any additional steps in marking textiles products. As a proof of concept, various woolen yarns and fabrics were finished using standard protocols and the survivability of the SigNature™ DNA was examined at the point of sale as described in the Examples below. In all textiles tested, SigNature™ DNA was recovered and the products were forensically authenticated. Thus, marking textile products with SigNature™ DNA can provide an economical, reliable, and secure method for marking, branding, and forensically authenticating textile products at the DNA level.

The disclosure also provides methods of binding an activated deoxyribonucleic acid or activated deoxyribonucleic acids to a plasma-treated substrate: The methods include exposing the deoxyribonucleic acid (DNA) to alkaline conditions, and contacting the alkaline-treated DNA to the plasma-treated substrate. The alkaline-treated DNA bound to the plasma-treated substrate is then available for binding by hybridization probes, and can be amplified by PCR techniques and the nucleotide sequence can be determined by DNA sequencing methods that depend on primer extension with the bound DNA as the template. Further examples of this method are disclosed in US20140256881.

As disclosed herein , the alkaline conditions are produced by mixing the deoxyribonucleic acid with an alkaline solution having a high pH, for instance the pH of the alkaline solution can be a pH of about 9.0 or higher; a pH of about 10.0 or higher; a pH of about 11.0 or higher, or even a pH of about 12.0 or higher, and contacting the deoxyribonucleic acid that has been exposed to the alkaline conditions with the substrate. In one example, the alkaline solution is a solution of a hydroxide of an alkali metal.

The present disclosure provides a method of binding a deoxyribonucleic acid to a plasma-treated substrate, the method includes exposing the deoxyribonucleic acid to alkaline conditions, wherein the alkaline conditions are produced by mixing the deoxyribonucleic acid with an alkaline solution, and contacting the deoxyribonucleic acid that has been exposed to the alkaline conditions with the substrate; wherein the alkaline solution is a solution of a hydroxide of an alkali metal and the alkali metal is selected from the group consisting of lithium (Li), sodium (Na), rubidium (Rb), and cesium (Cs).

The present disclosure provides a method of binding a deoxyribonucleic acid to a plasma-treated substrate, the method includes exposing the deoxyribonucleic acid to alkaline conditions, wherein the alkaline conditions are produced by mixing the deoxyribonucleic acid with an alkaline solution, and contacting the deoxyribonucleic acid that has been exposed to the alkaline conditions with the substrate; wherein the alkaline solution is a solution of an alkali metal hydroxide, wherein the alkali metal hydroxide is selected from the group consisting of lithium hydroxide (LiOH), sodium hydroxide (NaOH) and cesium hydroxide (CsOH). In one example, the alkali metal hydroxide is sodium hydroxide (NaOH).

The disclosure provides a method of binding a deoxyribonucleic acid to a plasma-treated substrate, the method including exposing the deoxyribonucleic acid to alkaline conditions, and contacting the deoxyribonucleic acid that has been exposed to the alkaline conditions with the plasma-treated substrate; wherein the alkaline conditions are produced by mixing the deoxyribonucleic acid with a solution of an alkali metal hydroxide, wherein the alkali metal hydroxide solution having a concentration of from about 1 mM to about 1.0 M.

The disclosure provides a method of binding a deoxyribonucleic acid to a plasma-treated substrate, the method including exposing the deoxyribonucleic acid to alkaline conditions and contacting the deoxyribonucleic acid that has been exposed to the alkaline conditions with the plasma-treated substrate; wherein the alkaline conditions are produced by mixing the deoxyribonucleic acid with a solution of an alkali metal hydroxide, the alkali metal hydroxide solution having a concentration of from about 10 mM to about 0.9 M.

The disclosure provides a method of binding a deoxyribonucleic acid to a plasma-treated substrate, the method including exposing the deoxyribonucleic acid to alkaline conditions and contacting the deoxyribonucleic acid that has been exposed to the alkaline conditions with the plasma-treated substrate; wherein the alkaline conditions are produced by mixing the deoxyribonucleic acid with a solution of an alkali metal hydroxide, the alkali metal hydroxide solution having a concentration of from about 0.1 M to about 0.8 M.

The disclosure provides a method of binding a deoxyribonucleic acid to a plasma-treated substrate, the method including exposing the deoxyribonucleic acid to alkaline conditions and contacting the deoxyribonucleic acid that has been exposed to the alkaline conditions with the plasma-treated substrate; wherein the alkaline conditions are produced by mixing the deoxyribonucleic acid with a solution of an alkali metal hydroxide, the alkali metal hydroxide solution having a concentration of about 0.6 M.

The disclosure provides a method of binding of a deoxyribonucleic acid to a plasma-treated substrate, wherein the method includes exposing the deoxyribonucleic acid to alkaline conditions and contacting the alkaline exposed deoxyribonucleic acid to the plasma-treated substrate, wherein the deoxyribonucleic acid is mixed with an alkaline solution having a pH from about 9.0 to about 14.0 and incubated at a temperature of from about 0°C to about 65°C to produce the alkaline conditions.

The disclosure provides a method of binding of a deoxyribonucleic acid to a plasma-treated substrate, wherein the method includes exposing the deoxyribonucleic acid to alkaline conditions and contacting the alkaline exposed deoxyribonucleic acid to the plasma-treated substrate, wherein the deoxyribonucleic acid is mixed with an alkaline solution having a pH from about 9.0 to about 14.0 and incubated at a temperature of from about 5°C to about 55°C to produce the alkaline conditions.

The disclosure provides a method of increasing binding of a deoxyribonucleic acid to a plasma-treated substrate, wherein the method includes exposing the deoxyribonucleic acid to alkaline conditions and contacting the alkaline exposed deoxyribonucleic acid to the plasma-treated substrate, wherein the deoxyribonucleic acid is mixed with an alkaline solution having a pH from about 9.0 to about 14.0 and incubated at a temperature of from about 10°C to about 45°C to produce the alkaline conditions.

The disclosure provides a method of increasing binding of a deoxyribonucleic acid to a plasma-treated substrate, wherein the method includes exposing the deoxyribonucleic acid to alkaline conditions and contacting the alkaline exposed deoxyribonucleic acid to the plasma-treated substrate, wherein the deoxyribonucleic acid is mixed with an alkaline solution having a pH from about 9.0 to about 14.0 and incubated at a temperature of from about 15°C to about 35°C to produce the alkaline conditions.

The disclosure provides a method of binding a deoxyribonucleic acid to a plasma-treated substrate, the method including exposing the deoxyribonucleic acid to alkaline conditions, and contacting the deoxyribonucleic acid that has been exposed to the alkaline conditions with the plasma-treated substrate; wherein the alkaline conditions are produced by mixing the deoxyribonucleic acid with a solution of an alkali metal hydroxide and incubating the mixture at a temperature of from about 0 °C to about 65 °C.

The disclosure provides a method of binding a deoxyribonucleic acid to a plasma-treated substrate, the method including exposing the deoxyribonucleic acid to alkaline conditions, and contacting the deoxyribonucleic acid that has been exposed to the alkaline conditions with the plasma-treated substrate; wherein the alkaline conditions are produced by mixing the deoxyribonucleic acid with a solution of an alkali metal hydroxide and incubating the mixture at a temperature of from about 15 °C to about 22 °C.

Alternatively, the disclosure provides a method of binding a deoxyribonucleic acid to a plasma-treated substrate, deoxyribonucleic acid can be mixed with a solution of any suitable high pH buffer to produce the alkaline conditions and contacting the deoxyribonucleic acid that has been exposed to the alkaline conditions with the plasma-treated substrate. The high pH buffer can be any suitable high pH buffer with a pKa in a range of from about 9.0 to about 11.0 or higher. In one example, the pH of the high pH buffer can be, for example, a pH of about 9.0 or higher; a pH of about 10.0 or higher; or a pH of about 11.0 or higher. For example, deoxyribonucleic acid can be mixed with a suitable high pH buffer such as CABS (4-[cyclohexylamino]-1-butanesulphonic acid) with a useful pH range of about 10.0-11.4 (at 25 °C) and a pKa of about 10.70 (at 25 °C) Product No. C5580 Sigma Aldrich, St. Louis, Missouri; CAPS (N-cyclohexyl-3-aminopropanesulfonic acid) with a useful pH range of about 9.7-11.1 (at 25 °C), a pKa of about 10.56 (at 20 °C), a pKa of about 10.40 (at 25 °C) and a pKa of about 10.02 (at 37 °C) Sigma Aldrich Product Nos. C6070 and C2632; AMP (2-amino-2-methyl-1-propanol) with a useful pH range of about 9.0-10.5 (at 25 °C), a pKa of about 9.70 (at 25 °C) Sigma Aldrich Product Nos. A9199 and A9879; CAPSO (*N*-cyclohexyl-2-hydroxyl-3-aminopropanesulfonic acid) with a useful pH range of about 8.9-10.3 (at 25 °C), a pKa of about 9.60 (at 25 °C), a pKa of about 9.43 (at 37 °C) Sigma Aldrich Product Nos. C2278 and C8085; CHES (2-(N cyclohexylamino)ethanesulphonic acid) with a useful pH range of about 8.60-10.0 (at 25 °C), a pKa of about 9.55 (at 20 °C), a pKa of about 9.49 (at 25 °C) and a pKa of about 9.36 (at 37 °C) Sigma Aldrich Product Nos. C2885 and C8210; AMPSO (3-[(1,1-dimethyl-2-hydroxyethyl)amino]-2-hydroxypropanesulfonic acid) with a useful pH range of about 8.3- 9.7 (at 25 °C), a pKa of about 9.00 (at 25 °C), a pKa of about 9.10 (at 37 °C) Sigma Aldrich Product Nos. A6659 and A7585, to produce the alkaline conditions.

In one example, the deoxyribonucleic acid that has been exposed to the alkaline conditions is added as a component of a liquid composition. The liquid composition any be any suitable liquid composition, such as for instance, a printing ink. For example, the ink may be a heat-curing epoxy-acrylate ink, such as Product No. 4408R or the 970 series Touch Dry® pellet each from Markem®, Keene, NH. Alternatively, the Artistri® P5000 + Series-Pigment Ink from Dupont®, or an Epoxy Acrylate Ink, such as Product No. 00-988, from Rahn USA Corp. can be used.

In one example, the taggant includes a nucleic acid. In one example, the taggant consists essentially of DNA and no other significant component useful for identification or authentication. Alternatively, or in addition, other taggants such as, for example, ultraviolet (UV) taggants, Up Converting Phosphor (UCP) infrared (IR) taggants, UV marker taggants, UV fluorophore taggants, ceramic IR marker taggants, protein taggants, and/or trace element taggants can be used in combination with deoxyribonucleic acid taggants activated by alkaline treatment according to the methods of the present disclosure. In one example, the taggants used may include, for example, a combination of DNA taggants, and an IR upconverting phosphor (UCP) taggant. In another example, the taggants used may include, for example, a combination of DNA taggants, an IR upconverting phosphor (UCP) taggant and a UV taggant. For example, the IR (UCP) taggant can be, for example, a green, a blue or a red (UCP) IR taggant, such as for instance the Green IR Marker, Product No. BPP-1069; the Blue UCP, Product No. BPP-1070; or the Red UCP, Product No. BPP-1071 from Boston Applied Technologies Inc., Woburn, MA.

The solution in which the soluble taggants are dissolved according to the methods of the present disclosure can include, for example, water, TE buffer (10 mM Tris·HCl, 1 mM EDTA), Tris-glycine buffer, Tris-NaCl buffer, TBE buffer (Tris-borate-EDTA), TAE buffer (Tris-acetate-EDTA) and TBS buffer (Tris-buffered saline), HEPES buffer (N-(2-Hydroxyethyl)piperazine-N'-ethanesulfonic acid), MOPS buffer (3-(N-Morpholino)propanesulfonic acid), PIPES buffer (Piperazine-N,N'-bis(2-ethanesulfonic acid), MES buffer (2-(N-Morpholino)ethanesulfonic acid), PBS (Phosphate Buffered Saline), PBP buffer (sodium phosphate + EDTA), TEN buffer (Tris/EDTA/NaCl), TBST buffer (Tris-HCl, NaCl, and Tween 20), PBST buffer (Phosphate Buffered Saline with Tween 20) and any of the many other known buffers used in the biological and chemical sciences.

The objects of interest marked with the deoxyribonucleic acid and optional additional taggants according to examples of the present disclosure include, for example, ceramic surfaces, plastic films, vinyl sheets, antiques, items of jewelry, identification cards, credit cards, magnetic strip cards, paintings, artwork, souvenirs, sports collectibles and other collectibles. The authenticity of these objects can then be verified by identifying the taggants bound or covalently bonded thereon by, for example, methods described in further detail below.

In one example, the surface to which the deoxyribonucleic acid that has been exposed to alkaline conditions is bound can be the surface of an object or item formed of a polymer, such as a polymer selected from the group consisting of polycarbonate (PC), polymethyl methacrylate (PMMA), polyurethane (PU), polystyrene (PS), nylon or polypropylene (PP) all of which are readily commercially available.

The disclosure provides methods for increasing the recoverability of a taggant from an object without disturbing the appearance of the object. Several examples of the present disclosure are described in detail below.

The present disclosure also provides methods for authenticating an object using taggants that have been incorporated onto an object or into a liquid for binding of an activated DNA taggant.

For example, the disclosure provides a method for increasing the recoverability of a taggant from an object; the method includes incorporating a DNA taggant onto the surface of an object or into a liquid for binding of the activated DNA taggant to an object or surface.

The present disclosure generally includes methods of binding DNA to treated surfaces, compounds formed by DNA bound to DNA treated surfaces, and systems for binding DNA to treated surfaces. Accordingly, while these methods are susceptible to various modifications and alternative forms, the specific methods disclosed herein are by way of the examples and in the drawings are for illustration only.

As discussed above, DNA is a polymer having a free hydroxyl group at the 3' terminus and a free phosphate group at the 5' terminus of each single strand. These groups can bind to exogenous reactive groups of other molecules. Without wishing to be bound by theory, it is believed that plasma treatment of the surface of an object to be marked with DNA produces reactive groups which bond the DNA applied to the treated surface. Such a reaction can be used to mark the surface of an object with DNA for identification or for forensic authentication.

A flowchart of a method according to the disclosure for binding DNA to a surface of an object is presented in FIG. 5. Referring now to the figure, at step 51, the surface is exposed to a plasma treatment to produce a plasma-treated surface. Atmospheric plasma can be generated inside a nozzle using a radio-frequency or microwave frequency electric field to ionize a gas into charged particles of a plasma, including positive ions and negatively charged electrons. Commercially available units for producing such plasmas include the low-pressure plasma systems and atmospheric plasma systems commercially available from several industrial suppliers, including Thierry Corporation (Detroit, MI); Enercon Industries, Corporation (Menomonee Falls, WI); and HBZ Helmholtz Zentrum Berlin (Berlin, Germany). In these experiments a Thierry Diener Plasma System Femto-Plasma Cleaner, an Enercon Plasma3™ or an Enercon Dyne-A-Mite™ IT Plasma Treater unit were employed.

These machines can generate a room temperature, atmospheric pressure plasma using a 600 watt, 150 KHz RF signal. Compressed gas is used to expel the charged particles through momentum transfer at a rate of about 50L/min to bombard the surface of an object. The line speed of the surface in the plasma surface treatment can be as high as 50 feet/min.

The bombardment of charged particles can remove oxide layers and etch metal surfaces, and create radicals from the surface, which disintegrate other surface molecules and evaporate the by-product molecules, thereby cleaning the surface. By controlling the pressure and the type of gas, the plasma radicals can also generate functional groups on the surface of the object inducing secondary reactions, such as intermolecular cross-linking to target molecules. For example, an argon plasma is used for cleaning, while in other examples, a compressed air plasma can be used to create functional groups. Under certain conditions, these functional groups can form a stable bond directly with other exogenous molecules such as DNA

Next, at step 52, DNA is applied to the treated surface of an object to mark it with DNA. Exemplary surfaces that can be treated according to the present invention include, but are not limited to, thread, wool, cotton, fabrics, currency, silver and copper. Objects that can be treated according to the present disclosure include, but are not limited to objects such as wafers, and microchips with exposed ceramic, plastic glass, epoxy, silicon and metal, copper surfaces. The list of objects that can be treated according to the present disclosure also includes diamond, gold, precious stones, wood, and glass.

An exemplary fabric is Pima and Giza cotton, also called extra long staple (ELS), considered to be one of the superior blends of cotton that is extremely durable and absorbent. Unlike the more common upland cotton, which is of the species *Gossypium hirsutum*, pima cotton is obtained from the *Gossypium barbadense* species.

The DNA useful in the practice of the present disclosure can be of any length. In one example, the DNA strand is from about 25 bases to about 10,000 bases in length. In another example, the DNA strand is from about 50 bases to about 5,000 bases in length. In still another example, the DNA strand is from about 75 bases to about 500 bases in length. Alternatively, in other examples, the DNA strand can be from about 25 to about 500 nucleotides in length, or about 30 to about 400 nucleotides in length, or about 40 to about 300; or even about 50 to about 200 nucleotides in length.

DNA useful for anti-counterfeiting and authentication is disclosed in U.S. Patent Publication No. 2010/0285985 of Liang, et al.. Liang *et al*., discloses a method of producing a plurality of security markers that includes providing a single DNA template, providing a pool of reverse template DNA (rtDNA) oligonucleotides complementary to the template, grouping primers in the pool of rtDNA oligonucleotides into a plurality of smaller subsets using combinatorial variation techniques, and generating a plurality of security markers from the plurality of smaller subsets of rtDNA oligonucleotides in the pool of rtDNA oligonucleotides, where each of the smaller subsets defines a distinct security marker. A single stranded DNA having a sequence of the distinct security marker can be used as the DNA to be applied to a treated surface according to the present disclosure .

According to the disclosure, the DNA can be applied to the treated surfaces using a pressurized canister, which may be automated or part of a robotic manufacturing process, or by manual spraying. In other examples, the DNA can applied onto the treated surface by swabbing the surface with a swab containing DNA, wiping with a fabric containing DNA, washing in a solution containing the DNA, dipping into a solution containing the DNA, or by application of DNA in a vapor, such as by a chemical vapor deposition (CVD) process. Binding of DNA to a treated surface can be achieved with DNA concentrations as low as one picogram per liter (10⁻¹²g/L) or even one femtogram per liter (10⁻¹⁵ g/L). DNA bound to a treated surface is highly resistant to washing.

DNA bound to a treated surface can be detected, extracted, analyzed, and identified. The ability to detect the extracted DNA allows for objects whose surfaces have been treated to be marked with DNA for subsequent identification and authentication. Referring again to FIG. 1, at step 53, DNA is extracted from a dried surface and is amplified using PCR.

After PCR amplification, at step 54, the DNA samples were analyzed using capillary electrophoresis. A PerkinElmer ABI Prism® 310 Genetic Analyzer with voltage set to 15 KV, current at 30 µA, temperature at 60°C, and laser power at 9.9 mW was used for the experiments described herein. Amplicons of the expected sizes amplified from the marker DNA used were detected in all samples tested regardless of which of the above-described methods was used to apply the marker DNA to the plasma-treated surface.

Plasma treatment was in a Thierry Plasma Chamber at 100% power for 10 seconds at ambient temperature with the vacuum set at 75% maximum. Results from representative experiments are shown in the figures and described below.

### EXAMPLE 1: Marker DNA Binding to Textiles

FIGS. 2A, 2B and 2C show the DNA detection results after capillary electrophoresis of amplification products from DNA extracts of DNA bound to plasma-treated, wool thread, wool fabric and cotton surfaces, respectively, according to the above-described methods. Each panel shows the fluorescence intensity versus DNA strand size, corresponding to the number of bases of the DNA strand.

### EXAMPLE 2: Washing of Marker DNA Bound to Textiles

To ascertain whether DNA was tightly bound to different surfaces after plasma treatment, wool yarn, wool fabric, cotton were treated with a plasma, and then subjected to various washes. Fabrics were washed with harsh detergents for at least 1 hour, then rinsed for at least 3 hours, under running water at 70°C. The exemplary, nonlimiting detergent used in this example was Kieralon Jet B available from BASF SE, used at a concentration of 12 g/L. Any suitable commercially available detergent can be used, e.g. Tide® 2X, available from Proctor & Gamble. After drying, DNA was PCR amplified and analyzed, as before. In all samples, DNA was detected before and after washes, demonstrating that with plasma treatment, DNA becomes tightly bound to the various surfaces. As a negative control, DNA was sprayed onto various surfaces that were not plasma treated. The surfaces were washed and the DNA was PCR amplified and analyzed. After water rinses, DNA was completely removed by washing, demonstrating that plasma treatment facilitates strong DNA binding to the various surfaces. The panels of Fig. 3A-3C show the results obtained.

FIG. 3A shows the detection results for plasma-treated wool yarn after DNA binding and washing. FIGS. 3B and 3C show the amplicon detection results from a similarly treated wool fabric before and after washing, respectively, that was not subjected to plasma pre-treatment. The DNA is detected after washing only of the surface that has been plasma-treated. Without plasma treatment of the surfaces to which the marker DNA was applied, washing was sufficient to remove the marker DNA and subsequent attempts at marker DNA detection were unsuccessful. These results demonstrate that plasma treatment renders binding of DNA to be resilient washing.

### EXAMPLE 3: Washing of Marker DNA Bound to a Microchip

FIG. 4A shows the DNA detection results obtained for DNA extracted from a microchip surface after DNA application to a plasma-treated surface. FIGS. 4B and 4C illustrate representative results of DNA detection from DNA extracted before and after washing both plasma-treated and non-treated metal microchip surfaces to which marker DNA had been applied. Microchips were rinsed with a strong flow of hot water for at least 1 minute. Excess water was sponged dry and the samples were dried further in a 60°C oven for at least 1 hour. FIG. 4B shows the detection results for a treated metal microchip before washing. FIG. 4C shows the detection results from the same microchip after washing.

### EXAMPLE 4: Washing of Marker DNA Bound to Plasma-treated Foil

FIGS. 5A and 5B illustrate representative results of marker DNA detection by PCR amplification in samples obtained before and after washing plasma-treated glass surfaces to which the marker DNA had been applied.

Results of capillary electrophoresis analysis of PCR amplifications of marker DNA bound to plasma-treated aluminum foil obtained from samples of foil clippings or washes in a first experiment are shown panels FIGS. 6A-6I.

Twenty microliters of a double-stranded marker DNA at a concentration of 2.5 ng/ml in deionized distilled water was evenly spread over the surface of an aluminum foil square of approximately 5 mm x 5 mm that had been plasma-treated as described above to yield an estimated marker DNA density of approximately 0.5 ng/mm². The solution was dried onto the surface of the plasma-treated foil under vacuum for 20 mins. A clipping from the middle of the foil of from about 0.5 mm to about 1.0 mm square was cut from the foil and used in PCR detection of the marker DNA. Capillary electrophoresis results are shown in Fig. 6A. The expected amplicon of 331 bases amplified from the marker DNA used in this experiment was detected. The remaining foil was washed under running hot water for about 1 min. and another clipping sample was taken and subjected to PCR. Again, the expected amplicon was detected as shown in Fig. 6B. The foil clipping was retrieved from the first wash and was then subjected to a series of washes in water as follows: At each wash the foil was placed in 50 ml deionized distilled water in a plastic Falcon tube and vortexed for -30 seconds to about 1 minute. A sample of 1 microliter of the wash water was then subjected to PCR analysis for the detection of marker DNA. The foil clipping from the first serial wash was retrieved and dried and washed in the second serial wash of 50 ml deionized distilled water as before. The third, fourth, fifth and sixth serial washes were performed as for the first and second washes. Figs. 6C-6H show the capillary electrophoresis results obtained for the first through the sixth washes. Marker DNA amplicons were detected in the first and second washes, but not in subsequent washes. The foil clipping after the sixth serial wash was then subjected to *in situ* PCR. The capillary electrophoresis results of the amplified products are shown in Fig. 6I. The amplicon from PCR amplification of marker DNA is clearly seen demonstrating that detectable the marker DNA was still bound to the foil after the hot water wash followed by the six serial washes.

### EXAMPLE 5: Washing of Marker DNA Bound to Untreated Foil

A second experiment was run with the same marker DNA spread over the surface of a similarly sized aluminum foil square that had not been subjected to plasma-treatment. Results obtained are shown in Figs. 7A-7I. This time the amplified 331 base amplicon was detected in each of the six washes following the hot water wash, demonstrating that the DNA continues to leach from the untreated foil in the serial washes. Without wishing to be bound by theory, these results suggest that the DNA is less tightly bound to the untreated foil than to the plasma-treated foil of the previous experiment.

### EXAMPLE 6: Recovery of Marker DNA Bound to a Ceramic/Plastic Portion and a Metallic Portion of a Microchip

A microchip was labeled on the metallic portion by spotting with marker DNA mixed with a fluorophore (F) in the top spot, or marker DNA with no fluorophore (nf) in the bottom spot. FIG. 8A. The DNA spots were dried under vacuum.

FIG. 8B shows the capillary electrophoresis results obtained from an amplification of an ethanol swab sample from a DNA spot on the plasma-treated plastic/ceramic portion of the microchip. FIG. 8C shows the capillary electrophoresis results obtained from an amplification of an ethanol swab sample from a DNA spot on the metallic portion of the microchip.

The fingertips of a latex glove were rubbed against the dried DNA spot and ethanol swab samples from the fingertips of the latex glove after rubbing the fingertips against the marker DNA spot on the plasma-treated microchip surface.

FIGS. 9A and 9B show the capillary electrophoresis results obtained. No Barely detectable DNA amplification was observed. The 331 base amplicon peak corresponding to the marker DNA detected after mechanically chafing or abrading the latex glove over the plasma treated surface of the microchip to which the marker DNA was bound suggests that the marker DNA was tightly bound to the microchip surface so that very little DNA was released by rubbing, chafing and abrading with the latex glove.

### EXAMPLE 7: Recovery of Marker DNA Bound to Copper Wire

The sheath was removed and coverings of the strands of a multi-core copper wire were stripped to expose half the length of the wire. The wire was plasma-treated in a Thierry Plasma Chamber at 100% power for 10 seconds at ambient temperature with the vacuum set at 75% maximum. FIG. 10A shows the partially exposed metal wire with a Teflon® sheath removed from a portion of the wire on a disposable bench pad measuring 10 cm x 10 cm. The exposed wire surface was estimated to cover approximately 0.5 % of the bench pad area.

Marker DNA was alkali-activated in a 0.6 M NaOH solution for 30 mins. at ambient temperature (about 18°C) and neutralized by dilution in buffer at neutral pH. In the first test, 1.5 ml volume containing 7.5 pg of the alkali-activated marker DNA solution was sprayed onto the wire segment and bench pad so that the DNA solution was substantially uniformly deposited over the entire 10 cm x 10 cm area. After drying in air, the wires and insulation were washed with Kieralon Jet B (BASF) although household detergent can be used. The wires and insulation were then thoroughly rinsed under running hot water for 3 minutes using the maximum water pressure from a regular laboratory sink faucet. FIG. 10B shows the capillary electrophoresis results obtained from amplification of a sample of the alkali activated DNA-treated exposed wire that had been subjected to plasma pretreatment. The 331 base amplicon corresponding to the PCR product after amplification of the marker DNA is clearly visible. Similar results were obtained with amplifications from samples of the wire coatings that had been sprayed with marker DNA.

In a parallel experiment alkali-activated marker DNA was sprayed on copper wire as described above, except that the wire was not subjected to plasma pretreatment. FIG. 10C shows the capillary electrophoresis results obtained from amplification of a sample of the alkali activated DNA-treated exposed wire that had no plasma pretreatment. The amplicon corresponding to the marker DNA was detected as before.

In a second parallel experiment marker DNA that had not been alkali-activated was sprayed on copper wire not subjected to plasma pretreatment, otherwise as described above. FIG. 10D shows the capillary electrophoresis results obtained from amplification of a sample of the marker DNA-treated exposed wire that had no plasma pretreatment. In this experiment no amplicon corresponding to the marker DNA was detected.

## Claims

1. A composition comprising alkaline-activated single stranded marker DNA bonded to a plasma-treated surface, wherein the composition is produced by applying alkaline-activated DNA to the plasma treated surface.

2. The composition of claim 1, wherein the plasma treatment comprises treatment with a plasma selected from an argon plasma, a compressed air plasma, a flame-based plasma and a vacuum plasma.

3. The composition of claim 1, wherein the surface is a surface of a ceramic, a semiconductor, a metal, a fabric or an organic polymer.

4. The composition of any preceding claim, wherein the DNA consists essentially of from 20 to 10,000 bases.

5. The composition of claim 4, wherein the DNA consists essentially of from 50 to 5,000 bases.

6. The composition of claim 5, wherein the DNA consists essentially of from 75 to 500 bases.

7. A method of binding DNA to a surface, wherein the method comprises the steps of:
exposing the surface to a plasma to produce a plasma-treated surface;
exposing DNA to alkaline conditions; and
applying the alkaline-activated DNA to the plasma-treated surface to produce surface bound DNA on the treated surface.

8. The method of claim 7, further comprising:
extracting a DNA sample from the plasma-treated surface and amplifying the extracted DNA sample to produce an amplified DNA sample;
wherein the amplified DNA sample is identified as the DNA applied to the plasma-treated surface.

9. The method of claim 8, wherein the extracted DNA sample is amplified using PCR.

10. The method of claim 7, wherein plasma-treated surface comprises reactive functional groups created on the surface by the plasma treatment.

11. The method of claim 7, wherein a concentration of DNA applied to the treated surface is at least one femtogram per liter (∼10⁻¹⁵g/L).

12. A system for binding DNA to a surface, comprising:
a plasma generator adapted to treating a surface with a plasma to produce a plasma-treated surface; and
an applicator containing alkaline-activated single-stranded marker DNA adapted to applying DNA to the plasma-treated surface to produce surface bound DNA on the plasma-treated surface.

13. The system of claim 12, wherein the DNA consists essentially of from 25 to 10,000 bases.

14. The system of claim 13, wherein the DNA consists essentially of from 50 to 5,000 bases.

15. The system of claim 14, wherein the DNA consists essentially of from 75 to 500 bases.

## Patentansprüche

1. Zusammensetzung, umfassend alkalisch aktivierte einzelsträngige Marker-DNA, die an eine plasmabehandelte Oberfläche gebunden ist, wobei die Zusammensetzung durch Anwenden alkalisch aktivierter DNA auf die plasmabehandelte Oberfläche hergestellt wird.

2. Zusammensetzung nach Anspruch 1, wobei die Plasmabehandlung eine Behandlung mit einem Plasma umfasst, das aus einem Argonplasma, einem Druckluftplasma, einem flammenbasierten Plasma und einem Vakuumplasma ausgewählt ist.

3. Zusammensetzung nach Anspruch 1, wobei die Oberfläche eine Oberfläche aus einer Keramik, einem Halbleiter, einem Metall, einem Gewebe oder einem organischen Polymer ist.

4. Zusammensetzung nach einem vorherigen Anspruch, wobei die DNA im Wesentlichen aus 20 bis 10.000 Basen besteht.

5. Zusammensetzung nach Anspruch 4, wobei die DNA im Wesentlichen aus 50 bis 5000 Basen besteht.

6. Zusammensetzung nach Anspruch 5, wobei die DNA im Wesentlichen aus 75 bis 500 Basen besteht.

7. Verfahren zum Binden von DNA an eine Oberfläche, wobei das Verfahren die folgenden Schritte umfasst:
Aussetzen der Oberfläche an ein Plasma, um eine plasmabehandelte Oberfläche herzustellen;
Aussetzen der DNA an alkalische Bedingungen; und
Anwenden der alkalisch aktivierten DNA auf die plasmabehandelte Oberfläche, um oberflächengebundene DNA auf der behandelten Oberfläche herzustellen.

8. Verfahren nach Anspruch 7, ferner umfassend:
Extrahieren einer DNA-Probe aus der plasmabehandelten Oberfläche und Amplifizieren der extrahierten DNA-Probe, um eine amplifizierte DNA-Probe herzustellen;
wobei die amplifizierte DNA-Probe als die DNA identifiziert wird, die auf die plasmabehandelte Oberfläche angewendet wird.

9. Verfahren nach Anspruch 8, wobei die extrahierte DNA-Probe unter Verwendung von PCR amplifiziert wird.

10. Verfahren nach Anspruch 7, wobei die plasmabehandelte Oberfläche reaktive funktionelle Gruppen umfasst, die auf der Oberfläche durch die Plasmabehandlung erzeugt werden.

11. Verfahren nach Anspruch 7, wobei eine Konzentration von DNA, die auf die behandelte Oberfläche angewendet wird, mindestens ein Femtogramm pro Liter (∼10-¹⁵g/l) ist.

12. System zum Binden von DNA an eine Oberfläche, umfassend:
einen Plasmagenerator, der angepasst ist, um eine Oberfläche mit einem Plasma zu behandeln, um eine plasmabehandelte Oberfläche zu erzeugen; und
einen Applikator, der alkalisch aktivierte einzelsträngige Marker-DNA enthält, die angepasst ist, um DNA auf die plasmabehandelte Oberfläche anzuwenden, um oberflächengebundene DNA auf der plasmabehandelten Oberfläche herzustellen.

13. System nach Anspruch 12, wobei die DNA im Wesentlichen aus 25 bis 10.000 Basen besteht.

14. System nach Anspruch 13, wobei die DNA im Wesentlichen aus 50 bis 5000 Basen besteht.

15. System nach Anspruch 14, wobei die DNA im Wesentlichen aus 75 bis 500 Basen besteht.

## Revendications

1. Composition comprenant de l'ADN marqueur simple brin à activation alcaline lié à une surface traitée au plasma, dans laquelle la composition est produite par application d'ADN à activation alcaline à la surface traitée au plasma.

2. Composition selon la revendication 1, dans laquelle le traitement au plasma comprend le traitement avec un plasma sélectionné parmi un plasma d'argon, un plasma d'air comprimé, un plasma à base de flamme et un plasma sous vide.

3. Composition selon la revendication 1, dans laquelle la surface est une surface d'une céramique, d'un semi-conducteur, d'un métal, d'un tissu ou d'un polymère organique.

4. Composition selon l'une quelconque des revendications précédentes, dans laquelle l'ADN est constitué essentiellement par 20 à 10 000 bases.

5. Composition selon la revendication 4, dans laquelle l'ADN est constitué essentiellement par 50 à 5 000 bases.

6. Composition selon la revendication 5, dans laquelle l'ADN est constitué essentiellement par 75 à 500 bases.

7. Procédé de liaison d'ADN à une surface, dans lequel le procédé comprend les étapes :
d'exposition de la surface à un plasma pour produire une surface traitée au plasma ;
d'exposition d'ADN à des conditions alcalines ; et
d'application d'ADN à activation alcaline à la surface traitée au plasma pour produire de l'ADN lié à une surface sur la surface traitée.

8. Procédé selon la revendication 7, comprenant en outre :
l'extraction d'un échantillon d'ADN de la surface traitée au plasma et l'amplification de l'échantillon d'ADN extrait pour produire un échantillon d'ADN amplifié ;
dans lequel l'échantillon d'ADN amplifié est identifié comme l'ADN appliqué à la surface traitée au plasma.

9. Procédé selon la revendication 8, dans lequel l'échantillon d'ADN extrait est amplifié en utilisant la réaction en chaîne par polymérase (PCR).

10. Procédé selon la revendication 7, dans lequel la surface traitée au plasma comprend des groupes fonctionnels réactifs créés sur la surface par le traitement au plasma.

11. Procédé selon la revendication 7, dans lequel une concentration d'ADN appliqué à la surface traitée est d'au moins un femtogramme par litre (environ ∼10⁻¹⁵g/L).

12. Système de liaison d'ADN à une surface, comprenant :
un générateur de plasma conçu pour traiter une surface avec un plasma pour produire une surface traitée au plasma ; et
un applicateur contenant de l'ADN marqueur simple brin à activation alcaline conçu pour appliquer de l'ADN à la surface traitée au plasma pour produire de l'ADN lié à la surface sur la surface traitée au plasma.

13. Système selon la revendication 12, dans laquelle l'ADN est constitué essentiellement par 25 à 10 000 bases.

14. Système selon la revendication 13, dans laquelle l'ADN est constitué essentiellement par 50 à 5 000 bases.

15. Système selon la revendication 14, dans laquelle l'ADN est constitué essentiellement par 75 à 500 bases.
